# EUROPÄISCHE PATENTANMELDUNG

(11) **EP 2 014 261 A1**
(43) Veröffentlichungstag der Anmeldung: **14.01.2009**
(21) Anmeldenummer: 08159747.8
(22) Anmeldetag: 04.07.2008
(51) Int. Cl.: A61F 2/36

(54) **Set zur Erstellung eines Offset-Resurfacing-Hüftgelenksimplantates**

(30) Priorität: 09.07.2007 DE 102007032583
(71) Anmelder: ESKA Implants GmbH & Co. KG, 23556 Lübeck (DE)
(72) Erfinder: Grundei, Dr. Hans, 23558, Lübeck (DE); Gerdesmeyer, Dr. Ludger, 24105, Kiel (DE)
(74) Vertreter: Fuchs

(57) **Zusammenfassung**

Es wird ein Set zur Erstellung eines Offset-Resurfacing-Hüftgelenksimplantates beschrieben. Es weist eine 1 bis 1,5 mm dicke metallische Schale (1) zum Einsatz in das natürliche, lediglich entknorpelte Acetabulum sowie eine metallische Kappe (2) zum Aufsatz auf den natürlichen, lediglich entknorpelten Hüftgelenkskopf, deren Wandung im Bereich der Basiskante (6) sich im Querschnitt gesehen von einer Stärke von 2 mm bis zu 6 mm stetig vergrößert, so dass sich in ihrer Außenform eine Exzentrizität ergibt, und darüber hinaus ein in die Acetabulums-Schale (1) einsetzbares Inlay (3) mit einer Materialstärke zwischen 2 bis 5 mm als Gleitpartner für die Hüftgelenkskopf-Kappe (2) auf.

## Beschreibung

Die Erfindung betrifft ein Set zur Erstellung eines Offset-Resurfacing-Hüftgelenksimplantates. Hierunter wird ein Oberflächenersatz für die natürlichen Gleit- oder Artikulationsflächen des Acetabulums und des Hüftgelenkskopfes verstanden.

In jüngster Zeit kommen verstärkt so genannte Kappenimplantate zur Anwendung, welche über den präparierten natürlichen Restgelenkkopf des Hüftgelenkes gesetzt werden und in dieser Lage dann fixiert werden können. Kappenimplantate bestehen aus einer in ihrer äußeren Form der natürlichen Gelenkkugel nachgebildeten Kappe, die auf einen (teil-)präparierten natürlichen Restgelenkkopf setzbar ist. Ein derartiges Implantat lässt sich aus dem so genannten Set zur Erstellung eines Armierungsimplantates gemäß der DE-C-102 18 801 erstellen.

Vorraussetzung für eine stabile Sekundärfixation ist stabiles Knochenmaterial des Restknochens. So wird in der schon erwähnten Druckschrift vorgeschlagen, an die Gelenkkopfkappe einen Zapfen anzukoppeln, der in eine entsprechende Ausfräsung im Schenkelhals gesetzt wird. Dieser Zapfen weist eine Oberfläche auf, welche mit einer dreidimensionalen offenmaschigen Raumnetzstruktur versehen ist, in welche und durch welche hindurch Knochentrabekel des umliegenden Knochenmaterials wachsen und für die stabile Sekundärfixation sorgen.

Es gibt jedoch Indikationen, bei denen man noch davon absehen kann, den Schenkhals auszufräsen, um so Platz für den Zapfen zu schaffen. Angeführt wird hier die so genannte Perthes-Calvä-Legg-Krankheit, die ein- oder beidseitig im Bereich der Femurkopfepiphyse aseptische Knochennekrosen hervorruft. Vor allem bei Jungen vom 4. bis 12. Lebensjahr tritt diese Krankheit auf (Pschyrembel, Klinisches Wörterbuch, 259. Auflage, 2002, Seite 1285). Eine Ausheilung ohne Deformierung ist zwar möglich, jedoch bleibt eine eventuelle Walzen- oder Pilzform des Schenkelkopfes mit Abplattung der Hüftgelenkspfanne, seltener Coxa Plana oder Arthrosis deformans zurück.

Eine weitere Indikation ist beispielsweise eine Zyste im Hüftgelenkskopf, die zu Oberflächendefekten des Gelenkkopfes führt.

Ganz generell kann eine Nekrose des Gelenkkopfes zu oberflächenhaften Defekten führen, die es aber immer noch nicht rechtfertigen, den Gelenkkopf vollständig zu resezieren und den Patienten mit einer Kurzstielendoprothese (EP 0 878 176) zu versorgen.

Grundsätzlich - und dies wurde in jüngerer Zeit verstärkt erkannt - ist es günstig, mit (Teil-)resektionen von Knochen so lange wie möglich zu zögern, um bei einem eventuell später notwendigen Revisionseingriff auf mehrere Stufen der endoprothetischen Versorgung zurückgreifen zu können, von der Kurzstielendoprothese bis zu klassischen Langstielendoprothese. Der Einsatz der letztgenannten Endoprothese erfordert die vollständige Resektion des Schenkelhalses.

Mit einem Resurfacing-Hüftimplantat gemäß der DE 10 2005 011 361 B4 ist ein Vorschlag gemacht worden, mit welchem die größtmögliche Flexibilität hinsichtlich eines Langzeiterhaltes des Implantates in situ ermöglicht wird, also eine weitergehende endoprothetische Versorgung hilft hinaus zu zögern.

Dies ist durch ein Set zur Erstellung eines Resurfacing-Hüftimplantates geschaffen worden, welches eine 1 bis 1,5 mm dicke metallische Schale zum zementierten Einsatz in das natürliche, lediglich entknorpelte Acetabulum sowie eine 1 bis 1,5 mm dicke metallische Kappe zum zementierten Aufsatz auf den natürlichen, lediglich entknorpelten Hüftgelenkkopf und darüber hinaus ein in die Acetabulums-Schale einsetzbares Inlay mit einer Materialstärke zwischen 2 bis 5 mm als Gleitpartner für die Hüftgelenkskopf-Kappe aufweist.

Sowohl das knöcherne Acetabulum als auch der knöcherne Hüftgelenkskopf werden dabei mit einem Formfräser bearbeitet, wie dies bei einer Implantation eines Hüftgelenkstotalersatzimplantes der Fall ist. Acetabulum und Gelenkkopf werden lediglich von Knorpel und Bindegewebe befreit, d.h. entknorpelt.

Dieses Implantat hat sich in der Praxis bewährt. Allerdings gibt es Indikationen, bei denen Kopffehlsteilungen ausgeglichen werden müssen. Dies kann nach einem Trauma der Fall sein oder aber bei einem fortgeschrittenen Verschleiß.

Aufgabe der vorliegenden Erfindung ist es nun, das vorerwähnte Set zur Erstellung eines Resurfacing-Hüftgelenkimplantes so weiter zu bilden, dass Fehlstellungen des Hüftgelenkskopfes ausgeglichen werden können.

Gelöst wird diese Aufgabe durch ein Set zur Erstellung eines Offset-Resurfacing-Hüftgelenksimplantates, welches - wie das vorerwähnte Set - eine 1 bis 1,5 mm dicke metallische Schale zum Einsatz in das natürliche, lediglich entknorpelte Acetabulum aufweist. Ebenfalls ist eine metallische Kappe zum Aufsatz auf den natürlichen, lediglich entknorpelten Hüftgelenkskopf Teil des Sets. Allerdings vergrößert sich deren Wandung im Bereich der Basiskante im Querschnitt gesehen von einer Stärke von 2 mm bis zu 6 mm stetig, so dass sich in ihrer Außenform damit eine Exzentrizität ergibt. Wie schon bei dem bekannten Set ist darüber hinaus ein in die Acetabulums-Schale einsetzbares Inlay mit eine Materialstärke zwischen 2 bis 5 mm als Gleitpartner für die Hüftgelenkkopfkappe Bestandteil des Sets.

Mit einer "Probekappe", die dieselbe Außenkontur wie das eigentliche Kappenimplantat aufweist, kann der Operateur durch Drehen auf dem Hüftgelenkskopf die Stellung der dann auf den Hüftgelenkskopf aufzubringenden metallischen Kappe ermitteln. Aufgrund der Exzentrizität der Außenform der Kappe kann der Operateur also mit der "Probekappe" einen Ausgleich der Hüftgelenkskopf-Fehlstellung simulieren. Das metallische Implantat wird dann in dieselbe als optimal befundene Position gebracht wie die "Probekappe".

Die metallische Kappe kann auf dem Hüftgelenkskopf zementiert werden. Es ist aber auch eine zementlos zu implantierende Kappe denkbar.

Gemäß einer vorteilhaften Weiterbildung ist proximal im Polbereich der Kappe exakt mittig ein Führungsstift vorgesehen, der in eine in den Hüftgelenkskopf einzubringende Bohrung setzbar ist.

Um die Kompensation der Hüftgelenkskopf-Fehlstellung dauerhaft zu gestalten, ist vorzugsweise vorgesehen, dass im Inneren der Hüftgelenkskopfkappe wenigstens zwei Antirotationselemente in das Kappeninnere hineinragen. Sie greifen dann nach der Implantation in den Knochen des Hüftgelenkskopfes hinein und verhindern so ein Drehen der Kappe auf dem Gelenkkopf. Die entsprechende "Probekappe" weist selbstverständlich keine Antirotationselemente auf, da sie sich ja auf dem Gelenkkopf zum Auffinden der optimalen Position drehen lassen können muss. -

Gemäß einer konkreten Ausführungsform sind die Antirotationselemente schildförmig ausgebildet. Alternativ können sie stiftförmig ausgebildet sein.

Die Erfindung wird anhand eines Ausführungsbeispiels gemäß der Zeichnungsfiguren näher erläutert. Hierbei zeigt:
- Fig. 1: schematisch das aus dem Set zusammengestellte Hüftgelenksimplantat mit der im Acetabulum fixierten Schale sowie mit der auf dem Gelenkkopf platzierten Kappe,

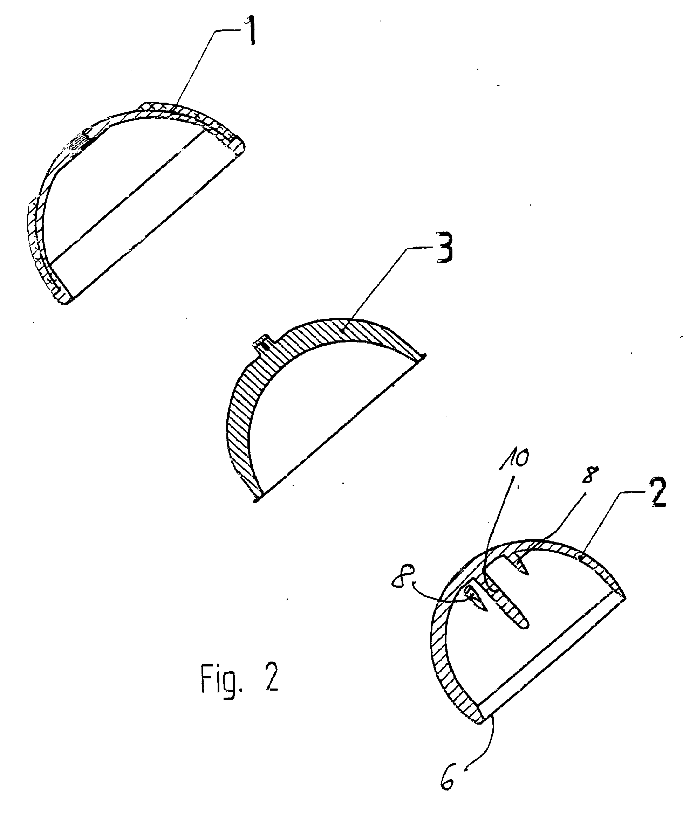

- Fig. 2: die Einzelteile des erfindungsgemäßen Sets,
- Fig. 3: eine Einsicht in das Innere der Kappe, und
- Fig. 4: eine schematische Schnittansicht durch die Kappe des Sets.

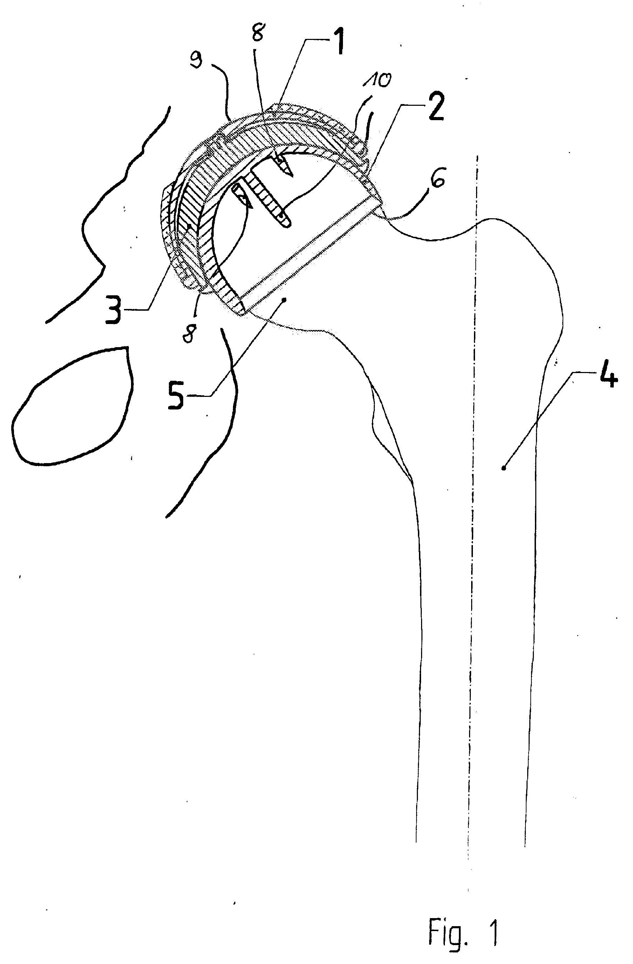

Fig. 1 veranschaulicht schematisch, wie das Hüftgelenksimplantat, das aus dem Set erstellt worden ist, implantiert ist. Auf den Hüftgelenkskopf 5 am Femur 4 ist die metallische Kappe 2 gesetzt. Sie kann dort mittels einer dünnen Zementschicht im Inneren der Kappe fixiert sein.

In das natürliche Acetabulum 9 ist die metallische Schale gesetzt und dort mit einer dünnen Zementschicht fixiert. Der verwendete Zement ist sehr dünnflüssig.

In die Schale 1 ist das Inlay 3 gesetzt, welches den Gleitpartner für die metallische Kappe 2 bildet. Im Inneren der Hüftgelenkskopf-Kappe 2 ist proximal im Polbereich exakt mittig ein Führungszapfen 10 angeformt, der in eine in den Schenkelkopfhals 5 einzubringende Bohrung greift. Darüber hinaus ragen im Inneren der Hüftgelenkskopf-Kappe 2 wenigstens zwei Antirotationselemente 8 in das Kappeninnere. Die Antirotationselemente stellen sicher, dass die einmal eingestellte Lage der Kappe 2 auf dem Gelenkkopf 5 langzeitstabil erhalten bleibt.

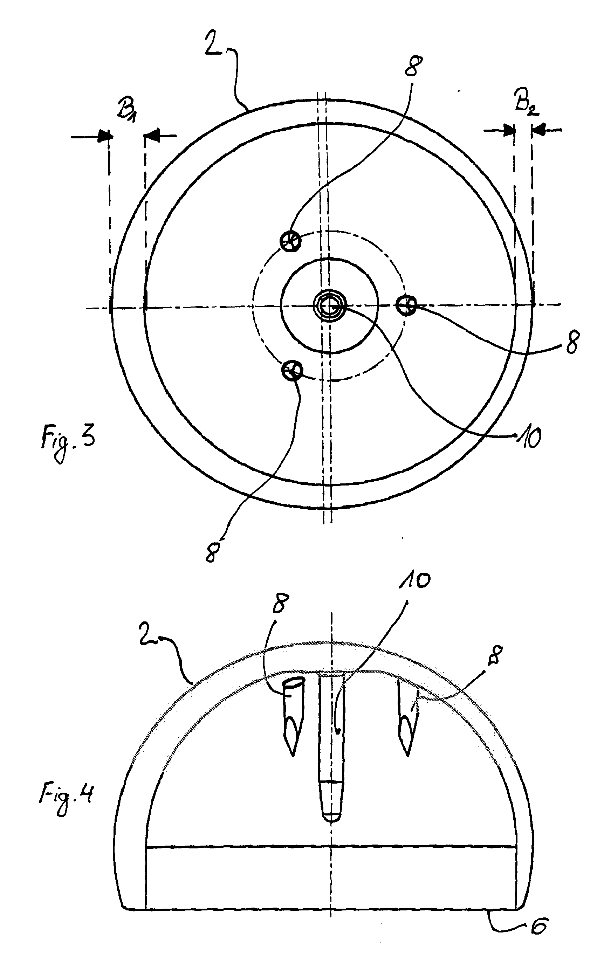

Das besondere an der Kappe ist nun, dass sich ihre Wandung im Bereich der Basiskante 6 im Querschnitt gesehen stetig vergrößert. Im dargestellten Ausführungsbeispiel ist die Wandung der Kappe 2 links größer als rechts. Dies wird noch deutlicher anhand der Figuren 3 und 4. Dort ist deutlich erkennbar, dass die Wandung links mit der Breite B₁ wesentlich breiter ist als auf der gegenüberliegenden Seite, wo die Wandung eine Breite B₂ aufweist. Aufgrund dieser Besonderheit ergibt sich eine Exzentrizität der Außenkontur der Kappe 2. Mit Hilfe dieser Exzentrizität ist es möglich, Fehlstellungen des Hüftgelenkskopfes 5 zu kompensieren. Durch Verdrehen einer "Probekappe", welche dieselben Außenkonturen aufweist wie das eigentliche Implantat, jedoch zumindest keine Antirotationselemente aufweist, kann die optimale Stellung des späteren Implantates in situ ermittelt werden. Die Wandung der Kappe 2 kann sich von 2 mm stetig bis auf 6 mm vergrößern.

## Patentansprüche

1. Set zur Erstellung eines Offset-Resurfacing-Hüftgelenksimplantates, aufweisend eine 1 bis 1,5 mm dicke metallische Schale (1) zum Einsatz in das natürliche, lediglich entknorpelte Acetabulum sowie eine metallische Kappe (2) zum Aufsatz auf den natürlichen, lediglich entknorpelten Hüftgelenkskopf, deren Wandung im Bereich der Basiskante (6) sich im Querschnitt gesehen von einer Stärke von 2 mm bis zu 6 mm stetig vergrößert, so dass sich in ihrer Außenform eine Exzentrizität ergibt, und darüber hinaus ein in die Acetabulums-Schale (1) einsetzbares Inlay (3) mit einer Materialstärke zwischen 2 bis 5 mm als Gleitpartner für die Hüftgelenkskopf-Kappe (2).

2. Set nach Anspruch 1 mit einem proximal im Polbereich der Kappe (2) exakt mittig mit dieser verbundenen Führungsstift (7).

3. Set nach Anspruch 1 oder 2, bei dem im Inneren der Hüftgelenkskopf-Kappe (2) wenigstens zwei Antirotationselemente (8) in das Kappeninnere hineinragen.

4. Set nach Anspruch 3, bei dem die Antirotationselemente (8) schildförmig ausgebildet sind.

5. Set nach Anspruch 3, bei dem die Antirotationselemente (8) stiftförmig ausgebildet sind.
